# EUROPÄISCHE PATENTSCHRIFT

(11) **EP 1 682 162 B1**
(45) Veröffentlichungstag und Bekanntmachung des Hinweises auf die Patenterteilung: **12.03.2008**
(21) Anmeldenummer: 04790853.8
(22) Anmeldetag: 26.10.2004
(51) Int. Cl.: A61K 36/33, A61K 31/135, A61P 25/24

(54) **VERWENDUNG VON FEIGENKAKTUS (OPUNTIA) ZUR HERSTELLUNG EINES MEDIKAMENTS ZUR BEHANDLUNG VON DEPRESSIONEN**
USE OF PRICKLY PEAR (OPUNTIA) FOR THE PREPARATION OF A MEDICAMENT FOR THE TREATMENT OF DEPRESSIONS
UTILISATION DE FIGUE DE BARBARIE (OPUNTIA) POUR LA PRÉPARATION D'UN MÉDICAMENT POUR LE TRAITMENT DES DEPRESSIONS

(30) Priorität: 28.10.2003 DE 10350194
(43) Veröffentlichungstag der Anmeldung: 26.07.2006
(73) Patentinhaber: Dr. Willmar Schwabe GmbH & Co. KG, 76227 Karlsruhe (DE)
(72) Erfinder: NÖLDNER, Michael, 76139 Karlsruhe (DE); SCHÖTZ, Karl, 76448 Durmersheim (DE)
(74) Vertreter: Adam, Holger
(86) Internationale Anmeldenummer: PCT/EP2004/012070
(87) Internationale Veröffentlichungsnummer: WO 2005/041994

(56) Entgegenhaltungen:
- WO-A-99/22724
- WO-A-03/037324
- HAN, Y.N. ET AL.: "Monoamine Oxidase B Inhibitors from the Fruits of Opuntia ficus-indica var. saboten" ARCHIVES OF PHARMACAL RESEARCH, Bd. 24, Nr. 1, Februar 2001 (2001-02), Seiten 51-54, XP008043723

## Beschreibung

Die Erfindung betrifft die Verwendung von Opuntien, Pflanzenteilen davon und/oder von daraus hergestellten Extrakten oder sonstigen Zubereitungen zur Herstellung eines Arzneimittels zur Behandlung oder zur Unterstützung der Behandlung von depressiven Verstimmungen und Erkrankungen oder von anderen affektiven Störungen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen ausgewählt aus Niedergeschlagenheit, Unlustgefühl, Melancholie, Antriebsschwäche, schwankender oder eingetrübter Stimmungslage oder Einschränkungen des emotionalen Wohlbefindens. Die Erfindung betrifft des Weiteren die Verwendung von Opuntien, Pflanzenteilen davon und/oder von daraus hergestellten Extrakten oder sonstigen Zubereitungen zur Herstellung eines diätetischen Nahrungsmittels.

Depressionen gehören mit einer 1-Jahres Prävalenzrate von ca. 10 Prozent und einer Lebens-Prävalenzrate von ca. 20 Prozent zu den häufigsten Erkrankungen in den westlichen Industrienationen. Sie beeinträchtigen den Patienten erheblich im privaten und beruflichen Bereich, irritieren seine Umgebung, belasten durch vermehrte Inanspruchnahme der ärztlichen Primärversorgung und vieler Ausfalltage aufgrund von Krankschreibungen ganz enorm unser Gesundheitssystem und sind im Einzelfall sogar tödlich. Schätzungen gehen heute davon aus, dass mehr als zwei Drittel der ca. 10.000 jährlichen Suizide in Deutschland auf Depressionen zurückgehen. Depressionen werden klinisch folgendermaßen eingeteilt (H.-J. Möller, Der Internist 41, 70 - 79 (2000)):
1. Endogene Depression, uni- und bipolar
2. Depressive Neurose
3. Reaktive Depression
4. Depression bei schizoaffektiver Psychose
5. Organisch bedingte Depression
6. Depressive Symptome bei Demenz

Darüber hinaus werden depressive Erkrankungen aufgrund des Ausprägungsgrades als leicht, mittelgradig oder schwer klassifiziert.

Vorstufen depressiver Erkrankungen können sich äußern in Niedergeschlagenheit, Unlustgefühl, Melancholie, Antriebsschwäche, schwankender oder eingetrübter Stimmungslage oder Einschränkungen des emotionalen Wohlbefindens.

Trotz einer Vielzahl unterschiedlicher Hypothesen sind die Ursachen depressiver Erkrankungen nur unzureichend geklärt. Zur medikamentösen Behandlung der Depression und anderer affektiver Störungen sind z. B. die im folgenden aufgeführten Arzneimittelgruppen geeignet (H.-J. Möller, Der Internist 41, 70 - 79 (2000)):
1. Selektive Serotonin-Wiederaufnahme-Hemmer (SSRI).
2. Selektive Noradrenalin-Wiederaufnahme-Hemmer (SNRI)
3. Selektive Serotonin und Noradrenalin-Wiederaufnahme-Hemmer (SSNRI).
4. Trizyklische Antidepressiva (TCA)
5. MAO-A-Hemmer
6. Sonstige synthetische Präparate
7. Phytopharmaka

All den hier aufgeführten Behandlungsverfahren ist gemein, dass sie, wenn überhaupt, erst nach einer Behandlungsdauer von 10 - 14 Tagen wirksam werden. Bei ca. 20 % der Patienten ist die medikamentöse Therapie auch bei Kombination verschiedener Präparate unzureichend. Darüber hinaus haben alle genannten Behandlungsverfahren den Nachteil, unerwünschte Nebenwirkungen zu verursachen, die häufig zum Abbruch der Therapie führen. Es besteht also ein erheblicher Bedarf, weitere Mittel zur Behandlung depressiver Erkrankungen und anderer affektiver Störungen und ihrer Vorstufen bereitzustellen, insbesondere solche Mittel, die einen oder mehrere der genannten Nachteile ganz oder teilweise überwinden.

Aufgabe der vorliegenden Erfindung ist es somit, derartige Mittel bereitzustellen.

Diese Aufgabe wird erfindungsgemäß gelöst durch die Verwendung von Opuntien, Pflanzenteilen davon und/oder von daraus hergestellten Extrakten oder sonstigen Zubereitungen, bevorzugt von Blüten von Opuntien und besonders bevorzugt von Blüten der Art Opuntia ficus-indica zur Herstellung eines diätetischen Nahrungsmittels sowie zur Herstellung eines Arzneimittels zur Behandlung oder zur Unterstützung der Behandlung von depressiven Verstimmungen und Erkrankungen oder von anderen affektiven Störungen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen, ausgewählt aus Niedergeschlagenheit, Unlustgefühl, Melancholie, Antriebsschwäche, schwankender oder eingetrübter Stimmungslage oder Einschränkungen des emotionalen Wohlbefindens sowie durch ein Arzneimittel zur Behandlung oder zur Unterstützung der Behandlung von depressiven Verstimmungen und Erkrankungen oder von anderen affektiven Störungen und ihrer Vorstufen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen, ausgewählt aus Niedergeschlagenheit, Unlustgefühl, Melancholie, Antriebsschwäche, schwankender oder eingetrübter Stimmungslage oder Einschränkungen des emotionalen Wohlbefindens, gekennzeichnet durch einen Gehalt an Opuntien, Pflanzenteilen davon und/oder an daraus hergestellten Extrakten oder sonstigen Zubereitungen, sowie durch eine pharmazeutische Zubereitung als orale Darreichungsform, die zudem geeignete pharmazeutisch verträgliche Hilfsstoffe enthält, wobei das Arzneimittel und die pharmazeutische Zubereitung zudem gekennzeichnet sind durch einen zusätzlichen Gehalt an einem oder mehreren Selektiven Serotonin- und Noradrenalin-Wiederaufnahme-Hemmer (SSNRI).

Extrakte aus Opuntienblüten werden verwendet zur Behandlung von Diarrhö, Prostatahyperplasie und Kolitis (British Herbal Pharmacopoeia 1983 published by the British Herbal Medicine Association's, S. 151 - 152). Extrakte aus Blättern und Blattsprossen (sog. Nopal) wirken beim Menschen Blutzucker senkend (A. C. Frati-Munari et al., Diabetes Care 11, 63 - 66 (1988) und A. C. Frati-Munari et al., Gac. Med. Mex. 128, 431 - 436 (1992)). In pharmakologischen Versuchen wurden für verschieden hergestellte Pflanzenzubereitungen ebenfalls Wirkungen gefunden. So zeigen alkoholische Extrakte des Stammes antiphlogistische, analgetische und antioxidative Effekte (Park et al., Arch. Pharm. Res. 21, 30 -34 (1998) und Fitoterapia 72, 288 - 290 (2001) und Lee et al., J. Agric. Food Chem. 50, 6490 - 6496 (2002)).

Weiterhin wurden diuretische und antiulcus Wirkungen an Ratten gezeigt (E. M. Galati et al., J. Ethnopharmacol. 79, 17 - 21 (2002) und E. M. Galati et al., J. Ethnopharmacol. 76, 1 - 9 (2001)). Für Früchte von Opuntia ficus-indica var. Saboten wurden MAO-B hemmende Effekte gezeigt (Han et al., Arch. Pharm. Res. 24, 51 - 54 (2001)). Ferner werden neuroprotektive Eigenschaften von Opuntia ficus-indica Extrakten beschrieben (Y. S. Lee et al., Korea Institute of Science and Technology, WO03/037324).

Überraschenderweise wurde festgestellt, dass Opuntien im Tierversuch deutlich antidepressiv wirksam sind. Eine derartige Wirkung ist bisher für Opuntien nicht beschrieben und war aufgrund der bisher bekannten pharmakologischen und klinischen Effekte nicht zu erwarten. Darüberhinaus wurde überraschenderweise festgestellt, dass Opuntien im Tierversuch die antidepressive Wirksamkeit von Venlafaxin potenzieren. Venlafaxin representiert den Prototyp der neuesten "Generation" von Antidepressiva (sogenannte SSNRI's).

Extrakte aus Opuntien können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemp. bis 100 °C unter gelinder bis heftiger Durchmischung innerhalb von 10 Min. bis 24 Std. unter Normaldruck bis zu 200 bar erhalten werden. Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck.

Das erfindungsgemäße Pflanzenmaterial bzw. die daraus hergestellten Extrakte können in Form von Pulvern, Granulaten, Tabletten, Dragees oder Kapseln oder auch als Lösung (z. B. Tee, Decoct) vorzugsweise oral verabreicht werden.

Zur Herstellung von Tabletten wird der Extrakt mit geeigneten pharmazeutisch verträglichen Hilfstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Das erfindungsgemäße Pflanzenmaterial bzw. die daraus hergestellten Extrakte können auch, ggf. unter Zusatz von Hilfstoffen wie z. B. Stabilisatoren, Füllmittel etc., in Kapseln abgefüllt werden. Die Dosierung erfolgt dabei so, dass pro Tag 5 bis 2000 mg, bevorzugt 10 bis 1000 mg, besonders bevorzugt 50 bis 500 mg Extrakt bzw. 50 mg bis 10 g, bevorzugt 0.5 bis 5 g getrocknetes Pflanzenmaterial zugeführt werden.

Im Fall von frischem (nicht oder teilweise getrocknetem) Pflanzenmaterial bezieht sich die oben genannte Menge auf den Trockenanteil.

Die Wirksamkeit von Opuntien bei depressiven Erkrankungen wird durch die nachstehend beschriebenen Versuche belegt.

Die antidepressive Wirksamkeit wurde mit dem sogenannten "Forced Swimming Test" an Ratten geprüft. Bei der Durchführung dieses Tests werden Ratten während einer definierten Zeit von 5 Minuten in eine für sie ausweglose Situation gebraucht (wassergefüllter Glaszylinder). Die Ratten reagieren in diesem Test mit einer als Immobilisationszeit bezeichneten Starre, die als Korrelat einer Depression interpretiert wird. Werden die Ratten vor Durchführung des Tests mit antidepressiv wirksamen Arzneimitteln behandelt, verringert sich die Immobilisationszeit. Da andere Psychopharmaka wie z.B. Anxiolytika oder Neuroleptika in diesem Test unwirksam sind, ist dieses Testsystem zum Nachweis antidepressiver Wirkungen gut geeignet (R. D. Porsolt et al., Eur. J. Pharmacol. 47, 379 - 391 (1978); R. D. Porsolt et al., Adv. Pharmacol. Sci., 137 - 159 (1991)). Alle bisher bekannten Antidepressiva müssen in diesem Test, ähnlich wie beim Patienten, über einen Zeitraum von mehreren Tagen verabreicht werden um wirksam zu sein. Die Versuchstiere wurden entweder mit der Prüfsubstanz, oder zu Kontrollzwecken nur mit dem Lösungsmittel oder zum Vergleich der Effektivität mit dem Tricyklischen Antidepressivum Imipramin behandelt. Imipramin wurde als Standard Vergleichssubstanz gewählt, weil es sowohl in der psychiatrischen Praxis, als auch im tierexperimentellen Modell eines der am stärksten wirkenden Antidepressiva ist.

Die Tabellen 1 bis 3 zeigen beispielhaft die Wirksamkeit von drei verschiedenen Opuntiaextrakten im Vergleich mit dem Tricyclischem Antidepressivum Imipramin, bestimmt nach einer Behandlungsdauer von 9 Tagen. Desweiteren wird in Tabelle 4 die Venlafaxin potenzierende Wirkung von Opuntiaextrakt gezeigt. In diesem Fall wurde die Hemmung bereits nach einer Behandlungsdauer von 3 Tagen ermittelt. Die Hemmung der Immobilität wurde in allen Tabellen zu Vergleichszwecken in Prozent - Hemmung gegen die Kontrollgruppe ausgedrückt.

**Tabelle 1**

| **Substanz** | **Dosis** | **Hemmung der Immobilität** |
|---|---|---|
| | **[mg/kg]** | **[%]** |
| Opuntia-Extrakt (Beispiel 1) | 10 | 16 |
| Opuntia-Extrakt (Beispiel 1) | 30 | 34 |
| Opuntia-Extrakt (Beispiel 1) | 100 | 52 |
| Opuntia-Extrakt (Beispiel 1) | 300 | 83 |
| Imipramin | 30 | 64 |

**Tabelle 2**

| **Substanz** | **Dosis** | **Hemmung der Immobilität** |
|---|---|---|
| | **[mg/kg]** | **[%]** |
| Opuntia-Extrakt (Beispiel 2) | 30 | 14 |
| Opuntia-Extrakt (Beispiel 2) | 100 | 33 |
| Opuntia-Extrakt (Beispiel 2) | 300 | 65 |
| Imipramin | 30 | 68 |

**Tabelle 3**

| **Substanz** | **Dosis** | **Hemmung der Immobilität** |
|---|---|---|
| | **[mg/kg]** | **[%]** |
| Opuntia-Extrakt (Beispiel 3) | 100 | 23 |
| Imipramin | 20 | 44 |

**Tabelle 4**

| **Substanz** | **Dosis** | **Hemmung der Immobilität** |
|---|---|---|
| | **[mg/kg]** | **[%]** |
| Opuntia-Extrakt (Beispiel 1) | 200 | 43 |
| Venlafaxin | 100 | 25 |
| Opuntia-Extrakt (Beispiel 1) + | 200 | 61 |
| Venlafaxin | 100 | |

### Beispiel 1: Trockenextrakt (Extraktionsmittel 60 Gew.-% EtOH) aus Blüten von Opuntia ficus-indica

200 g fein gemahlene Droge werden 2 mal mit je 1400 g 60 Gew % EtOH jeweils 1 Std. bei 60 °C gerührt, anschließend die Suspension über eine Fritte P4 abgesaugt, die vereinigten Filtrate im Vakuum bei 40 °C vom EtOH befreit, der verbliebene wässrige Rückstand eingefroren und lyophilisiert. Der erhaltene Feststoff wird im Vakuum bei 40 °C über P₂O₅ und KOH getrocknet: 30.2 g (15.1 %) Trockenextrakt.

### Beispiel 2: Trockenextrakt (wässriger Auszug) aus Blüten von Opuntia ficus-indica (Decoct)

200 g gemahlene Blüten werden mit 1.5 L siedendem Wasser übergossen, kurz umgerührt und 10 Min. belassen. Dann wird die voluminöse Masse über eine Fritte P4 filtriert, der Feststoff nochmals mit 1 L kochendem Wasser übergossen, die Masse nach 10 Min. über eine Fritte P4 abgesaugt, der verbliebene voluminöse Rückstand zentrifugiert, die abdekantierte Lösung mit den Filtraten vereinigt und gefriergetrocknet. Anschließend wird der Rückstand gemörsert und 24 Std. im Vakuum getrocknet 23.4 g (11.7 %).

### Beispiel 3: Trockenextrakt (Extraktionsmittel 96 Gew.-% EtOH) aus jungen Blattsprossen von Opuntia ficus-indica

12.0 kg frische, junge Blattsprossen von Opuntia ficus-indica werden zerkleinert und mit 16 kg 96 %igem Ethanol versetzt. Die Mischung wird 10 min bei Raumtemperatur mit einem Ultraturrax homogenisiert, über eine Nutsche vom groben Pflanzenmaterial befreit und das trübe Filtrat über einen Seitzfilter 1500 gegeben. Nun wird die klare Lösung im Vakuum bei 40 °C zum Spissum eingeengt und die zähe Masse bei 40 °C im Vakuum im Trockenschrank getrocknet: 248 g (2.1 % entsprechend 13.8 % berechnet auf einen Trockenanteil des Pflanzenmaterials von 15 %).

### Beispiel 4: Tabletten

Ein Trockenextrakt aus Opuntia ficus-indica (Beispiel 1) wird mit Hilfsstoffen gemischt und zu Tabletten verpresst (Tablettenkern = Position 1 - 6). Die Tabletten werden mit einem Überzug aus Hydroxypropylmethylcellulose versehen (Position 7 - 10).

| | **Bestandteil** | **mg/Tablette** |
|---|---|---|
| 1 | Trockenextrakt aus Opuntia ficus-indica | 100.0 |
| 2 | Mikrokristalline Cellulose | 117.0 |
| 3 | Laktose Monohydrat | 58.0 |
| 4 | Croscarmellose | 15.0 |
| 5 | Hochdisperses Siliciumdioxid | 3.0 |
| 6 | Magnesiumstearat | 6.0 |
| 7 | Hydroxypropylmethylcellulose | 15.0 |
| 8 | Polyethylenglycol | 3.0 |
| 9 | Talkum | 1.0 |
| 10 | Titandioxid | 2.0 |

## Patentansprüche

1. Verwendung von Opuntien, Pflanzenteilen davon und/oder von daraus hergestellten Extrakten oder sonstigen Zubereitungen zur Herstellung eines Arzneimittels zur Behandlung oder zur Unterstützung der Behandlung von depressiven Verstimmungen und Erkrankungen oder von anderen affektiven Störungen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen, ausgewählt aus Niedergeschlagenheit, Unlustgefühl, Melancholie, Antriebsschwäche, schwankender oder eingetrübter Stimmungslage oder Einschränkungen des emotionalen Wohlbefindens.

2. Verwendung von Opuntien, Pflanzenteilen davon und/oder von daraus hergestellten Extrakten oder sonstigen Zubereitungen zur Herstellung eines diätetischen Nahrungsmittels.

3. Verwendung nach Anspruch 1 oder 2, wobei die Pflanzenteile Blüten und/oder Blattsprossen von Opuntien sind.

4. Verwendung nach einem der Ansprüche 1 bis 3, wobei die Opuntien der Art Opuntia ficus-indica angehören.

5. Verwendung nach einem der Ansprüche 1 bis 4, wobei Extrakte von Opuntien oder deren Pflanzenteile zum Einsatz kommen.

6. Verwendung nach einem der Ansprüche 2 bis 5, wobei das diätetische Nahrungsmittel geeignet ist zur Behandlung oder zur Unterstützung der Behandlung von depressiven Verstimmungen und Erkrankungen oder von anderen affektiven Störungen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen, ausgewählt aus Niedergeschlagenheit, Unlustgefühl, Melancholie, Antriebsschwäche, schwankender oder eingetrübter Stimmungslage oder Einschränkungen des emotionalen Wohlbefindens.

7. Arzneimittel zur Behandlung von depressiven Verstimmungen und Erkrankungen oder von anderen affektiven Störungen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen, ausgewählt aus Niedergeschlagenheit, Unlustgefühl, Melancholie, Antriebsschwäche, schwankender oder eingetrübter Stimmungslage oder Einschränkungen des emotionalen Wohlbefindens, **gekennzeichnet durch** einen Gehalt an Opuntien, Pflanzenteilen davon und/oder an daraus hergestellten Extrakten oder sonstigen Zubereitungen und **durch** einen zusätzlichen Gehalt an einem oder mehreren Selektiven Serotonin- und Noradrenalin-Wiederaufnahme-Hemmer (SSNRI).

8. Arzneimittel gemäß Anspruch 7, wobei der SSNRI Venlafaxin ist.

9. Pharmazeutische Zubereitung, bestehend aus Opuntien, Pflanzenteilen davon und/oder aus daraus hergestellten Extrakten oder sonstigen Zubereitungen und geeigneten Hilfsstoffen als orale Darreichungsform, **gekennzeichnet durch** einen zusätzlichen Gehalt an einem oder mehreren Selektiven Serotonin- und Noradrenalin-Wiederaufnahme-Hemmer (SSNRI).

10. Pharmazeutische Zubereitung gemäß Anspruch 9, wobei der SSNRI Venlafaxin ist.

11. Verwendung einer Wirkstoffkombination, bestehend aus Opuntien, Pflanzenteilen davon und/oder aus daraus hergestellten Extrakten oder sonstigen Zubereitungen und aus einem oder mehreren Selektiven Serotonin- und Noradrenalin-Wiederaufnahme-Hemmer (SSNRI) zur Herstellung eines Arzneimittels zur Behandlung von depressiven Verstimmungen und Erkrankungen oder von anderen affektiven Störungen, die durch Antidepressiva beeinflussbar sind, ausgewählt aus Angst- und Panikstörungen, Bipolaren Depressionen, Somatisierungsstörungen und dem prämenstruellen Syndrom sowie von Vorstufen derartiger Erkrankungen, ausgewählt aus Niedergeschlagenheit, Unlustgefühl, Melancholie, Antriebsschwäche, schwankender oder eingetrübter Stimmungslage oder Einschränkungen des emotionalen Wohlbefindens.

12. Verwendung gemäß Anspruch 11, wobei der SSNRI Venlafaxin ist.

## Claims

1. Use of opuntias, plant parts thereof and/or extracts or other preparations produced therefrom for the manufacture of a medicament for the treatment or for supporting the treatment of episodes of depression and depressive diseases or other affective disorders which can be influenced by anti-depressive agents, selected for anxiety and panic disorders, bipolar depressions, somatization disorders and the premenstrual syndrome, as well as preliminary stages of such diseases, selected from abjectness, listlessness, melancholia, anergy, labile or saddened mood or limitations of the emotional well-being.

2. Use of opuntias, plant parts thereof and/or extracts or other preparations produced therefrom for the manufacture of a dietetic food product.

3. Use according to claim 1 or 2, wherein the plant parts are flowers and/or leaf sprouts of opuntias.

4. Use according to any one of claims 1 to 3, wherein the opuntias belong to the species opuntia ficus-indica.

5. Use according to any one of claims 1 to 4, wherein extracts of opuntias or plant parts thereof are employed.

6. Use according to any one of claims 2 to 5, wherein the dietetic food product is suitable for the treatment or for supporting the treatment of episodes of depression and depressive diseases or other effective disorders which can be influenced by anti-depressive agents, selected from anxiety and panic disorders, bipolar depressions, somatization disorders and the premenstrual syndrome, as well as preliminary stages of such diseases, selected from abjectness, listlessness, melancholia, anergy, labile or saddened mood or limitations of the emotional well-being.

7. Medicament for the treatment of episodes of depression and depressive diseases or other affective disorders which can be influenced by anti-depressive agents, selected from anxiety and panic disorders, bipolar depressions, somatization disorders and the premenstrual syndrome, as well as preliminary stages of such diseases, selected from abjectness, listlessness, melancholia, anergy, labile or saddened mood or limitations of the emotional well-being, **characterised by** a content of opuntias, plant parts thereof and/or extracts or other preparations produced therefrom and by an additional content of one or more selective serotonin and noradrenaline reuptake inhibitor (SSNRI).

8. Medicament according to claim 7, wherein the SSNRI is venlafaxine.

9. Pharmaceutical preparation consisting of opuntias, plant parts thereof and/or extracts or other preparations produced therefrom and suitable adjuvants as an oral administration form, **characterised by** an additional content of one or more selective serotonin and noradrenaline reuptake inhibitors (SSNRI).

10. Pharmaceutical preparation according to claim 9, wherein the SSNRI is venlafaxine.

11. Use of a combination of active ingredients, consisting of opuntias, plant parts thereof and/or extracts or other preparations produced therefrom and one or more selective serotonin and noradrenaline reuptake inhibitors (SSNRI) for the manufacture of a medicament for the treatment of episodes of depressions and depressive diseases or other affective disorders which can be influenced by anti-depressive agents, selected from anxiety and panic disorders, bipolar depressions, somatization disorders and the premenstrual syndrome, as well as preliminary stages of such diseases, selected from abjectness, listlessness, melancholia, anergy, labile or saddened mood or limitations of the emotional well-being.

12. Use according to claim 11, wherein the SSNRI is venlafaxine.

## Revendications

1. Utilisation de figues de Barbarie, de parties de la plante et/ou d'extraits préparés à partir de celle-ci ou d'autres compositions pour la préparation d'un médicament pour le traitement ou pour le soutien du traitement d'irritations et de maladies dépressives ou d'autres troubles affectifs, qui peuvent être influencés par des antidépresseurs, choisis parmi les troubles de l'angoisse et de panique, les troubles dépressifs bipolaires, les troubles de la somatisation et le syndrome prémenstruel, ainsi que des stades préliminaires de ces maladies choisis parmi la déprime, le sentiment d'ennui, la mélancolie, l'indolence, l'humeur variable ou la mauvaise humeur ou des limitations du ressenti émotionnel.

2. Utilisation de figues de Barbarie, de parties de la plante et/ou d'extraits préparés à partir de celle-ci ou d'autres compositions pour la préparation d'un aliment diététique.

3. Utilisation selon la revendication 1 ou 2, où les parties de plantes sont les fleurs et/ou jeunes feuilles de figue de Barbarie.

4. Utilisation selon une des revendications 1 à 3, où la figue de Barbarie appartient au type *Opuntia ficus-indica*.

5. Utilisation selon une des revendications 1 à 4, où l'on met en oeuvre des extraits de figue de Barbarie ou leurs parties de plante.

6. Utilisation selon une des revendications 2 à 5, où l'aliment diététique est approprié pour le traitement ou pour le soutien du traitement d'irritations et de maladies dépressives ou d'autres troubles affectifs, qui peuvent être influencés par des antidépresseurs, choisis parmi les troubles de l'angoisse et de panique, les troubles dépressifs bipolaires, les troubles de la somatisation et le syndrome prémenstruel, ainsi que des stades préliminaires de ces maladies choisis parmi la déprime, le sentiment d'ennui, la mélancolie, l'indolence, l'humeur variable ou la mauvaise humeur ou des limitations du ressenti émotionnel.

7. Médicament pour le traitement d'irritations et de maladies dépressives ou d'autres troubles affectifs, qui peuvent être influencés par des antidépresseurs, choisis parmi les troubles de l'angoisse et de panique, les troubles dépressifs bipolaires, les troubles de la somatisation et le syndrome prémenstruel, ainsi que des stades préliminaires de ces maladies choisis parmi la déprime, le sentiment d'ennui, la mélancolie, l'indolence, l'humeur variable ou la mauvaise humeur ou des limitations du ressenti émotionnel, **caractérisé par** une teneur en figue de Barbarie, parties de plante et/ou extraits préparés à partir de celle-ci ou autres compositions et par une teneur supplémentaire en un ou plusieurs inhibiteurs sélectifs des récepteurs de la sérotonine et de la noradrénaline (SSNRI).

8. Médicament selon la revendication 7, où le SSNRI est la venlafaxine.

9. Composition pharmaceutique, consistent en la figue de Barbarie, des parties de plante et/ou extraits préparés à partir de celle-ci ou autres compositions, et des auxiliaires appropriés en tant que forme d'administration orale, **caractérisée par** une teneur supplémentaire en un ou plusieurs inhibiteurs sélectifs des récepteurs de la sérotonine et de la noradrénaline (SSNRI).

10. Composition pharmaceutique selon la revendication 9, où le SSNRI est la venlafaxine.

11. Utilisation d'une combinaison d'agents actifs, consistant en des figues de Barbarie, des parties de plante et/ou extraits préparés à partir de celle-ci ou autres compositions, et en un ou plusieurs inhibiteurs sélectifs des récepteurs de la sérotonine et de la noradrénaline (SSNRI), pour la préparation d'un médicament pour le traitement de maladies dépressives ou d'autres troubles affectifs, qui peuvent être influencés par des antidépresseurs, choisis parmi les troubles de l'angoisse et de panique, les troubles dépressifs bipolaires, les troubles de la somatisation et le syndrome prémenstruel, ainsi que des stades préliminaires de ces maladies choisis parmi la déprime, le sentiment d'ennui, la mélancolie, l'indolence, l'humeur variable ou la mauvaise humeur ou des limitations du ressenti émotionnel.

12. Utilisation selon la revendication 11, où le SSNRI est la venlafaxine.
